# DEMANDE DE BREVET EUROPEEN

(11) **EP 1 568 684 A1**
(43) Date de publication de la demande: **31.08.2005**
(21) Numéro de dépôt: 05290421.6
(22) Date de dépôt: 24.02.2005
(51) Int. Cl.: C07C 217/76, A61K 7/13

(54) **Para-phénylènediamines secondaires portant un groupe alcoxy, composition tinctoriale les comprenant, procédés et utilisations**

(30) Priorité: 27.02.2004 FR 0402015
(71) Demandeur: L'OREAL, 75008 Paris (FR)
(72) Inventeur: Sabelle, Stéphane, 75005 Paris (FR)
(74) Mandataire: Dossmann, Gérard

(57) **Abrégé**

La présente demande concerne de nouvelles para-phénylènediamines secondaires portant un groupe alcoxy, un procédé permettant leur préparation, leur utilisation en coloration d'oxydation capillaire, une composition pour la teinture des fibres kératiniques, et en particulier les fibres kératiniques humaines telles que les cheveux, contenant, dans un milieu approprié pour la teinture, au moins une telle para-phénylènediamine secondaire, un procédé de teinture des fibres kératiniques, l'utilisation de cette composition en coloration capillaire, ainsi qu'un "kit" de teinture.

## Description

La présente demande concerne une nouvelle famille de para-phénylènediamines secondaires portant un groupe alcoxy, leur préparation et leur utilisation en coloration d'oxydation capillaire.

Il est connu de teindre les fibres kératiniques et en particulier les cheveux humains avec des compositions tinctoriales contenant des précurseurs de colorant d'oxydation, appelés généralement bases d'oxydation, tels que des ortho ou para-phénylènediamines, des ortho ou para-aminophénols et des composés hétérocycliques. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration, ces derniers étant choisis notamment parmi les méta-diaminobenzènes aromatiques, les méta-aminophénols, les méta-diphénols et certains composés hétérocycliques tels que des composés indoliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs tels que la lumière, les intempéries, le lavage, les ondulations permanentes, la transpiration et les frottements.

Les colorants doivent également permettre de couvrir les cheveux blancs, et être enfin les moins sélectifs possibles, c'est-à-dire permettre d'obtenir des écarts de coloration les plus faibles possibles tout au long d'une même fibre kératinique, qui est en général différemment sensibilisée (c'est-à-dire abîmée) entre sa pointe et sa racine.

De manière surprenante et avantageuse, la Demanderesse vient de découvrir qu'il est possible d'obtenir une nouvelle famille de para-phénylènediamines secondaires portant un groupe alcoxy capable de conduire à des colorations aux nuances variées, puissantes, esthétiques, peu sélectives et résistant bien aux diverses agressions que peuvent subir les fibres. La présente demande porte également sur un procédé de préparation de ces para-phénylènediamines secondaires portant un groupe alcoxy, ainsi que sur leur utilisation en coloration d'oxydation capillaire.

Un autre objet de l'invention concerne des compositions pour la teinture des fibres kératiniques, en particulier des fibres kératiniques humaines telles que les cheveux, comprenant au moins une para-phénylènediamine secondaire portant un groupe alcoxy permettant d'obtenir des colorations présentant les avantages précédemment mentionnés. En outre, ces compositions présentent un bon profil toxicologique.

L'invention a aussi pour objet un procédé de teinture mettant en oeuvre cette composition, l'utilisation de la composition selon la présente invention pour la teinture des fibres kératiniques, en particulier les fibres kératiniques humaines telles que les cheveux et un dispositif à plusieurs compartiments ou "kit" de teinture.

La composition de la présente invention permet en particulier d'obtenir une coloration de fibres kératiniques très puissante, peu sélective et tenace, en particulier à la lumière, tout en évitant la dégradation de ces fibres.

D'autres caractéristiques, aspects, objets et avantages de la présente invention apparaîtront encore plus clairement à la lecture de la description et des exemples qui suivent.

Les nouvelles para-phénylènediamines secondaires selon la présente invention sont des composés de formule générale (I) : 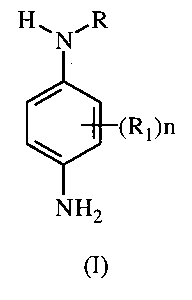 dans laquelle :
R représente un radical (alcoxy en (C₁-C₁₀) linéaire)alkyle en (C₄-C₁₀) linéaire, un radical (alcoxy en (C₁-C₁₀) linéaire)alkyle en (C₃-C₁₀) ramifié, un radical (alcoxy en (C₃-C₁₀) ramifié)alkyle en (C₂-C₁₀), un radical (alcoxy en (C₁-C₁₀))alkyle en (C₂-C₁₀) linéaire ou ramifié substitué par au moins un groupement hydroxy, alcoxy en C₁-C₁₅, amino, monoalkyl(C₁-C₁₅)amino, dialkyl(C₁-C₁₅)amino, alkyl(C₁-C₁₅)carbonyle, amido, alcoxy(C₁-C₁₅)carbonyle, mono ou dialkyl(C₁-C₁₅)amino carbonyle;
R₁ représente un atome d'hydrogène, un radical alkyle en C₁-C₁₅, un alcoxy en C₁-C₁₅, un (alcoxy en (C₁-C₁₅))alkyle en C₁-C₁₅, un hydroxyalcoxy en C₁-C₁₅, un (mono ou poly)hydroxy alkyle en C₁-C₁₅, ou un atome d'halogène;
n représente un entier variant de 1 à 4 ;
ou ses sels d'addition ;
à l'exception des composés suivants :
N-(3-isopropoxypropyl)-benzène 1,4-diamine;
N-(1-méthylpropoxypropyl)-benzène 1,4-diamine.
De préférence, le groupement R dans la formule (I) représente un radical (alcoxy en (C₁-C₅) linéaire)alkyle en (C₄-C₈) linéaire, un radical (alcoxy en (C₁-C₅) linéaire)alkyle en (C₃-C₇) ramifié, un radical (alcoxy en (C₃-C₇) ramifié)alkyle en (C₂-C₆), un radical (alcoxy en (C₁-C₆))alkyle en (C₂-C₆) linéaire ou ramifié substitué par au moins un groupement hydroxy, alcoxy en C₁-C₅, amino, monoalkyl(C₁-C₅)amino, alkyl(C₁-C₅)carbonyle, amido, alcoxy(C₁-C₅)carbonyle, mono ou dialkyl(C₁-C₅)amino carbonyle; et le groupement R₁ de formule (I) représente un atome d'hydrogène, un radical alkyle en C₁-C₅, un alcoxy en C₁-C₅, un (alcoxy en (C₁-C₅))alkyle en C₁-C₅, un hydroxyalcoxy en C₁-C₅, un (mono ou poly)hydroxy alkyle en C₁-C₅, ou un atome de chlore ou de brome.
De manière plus préférentielle, les composés de formule générale (I) sont choisis parmi

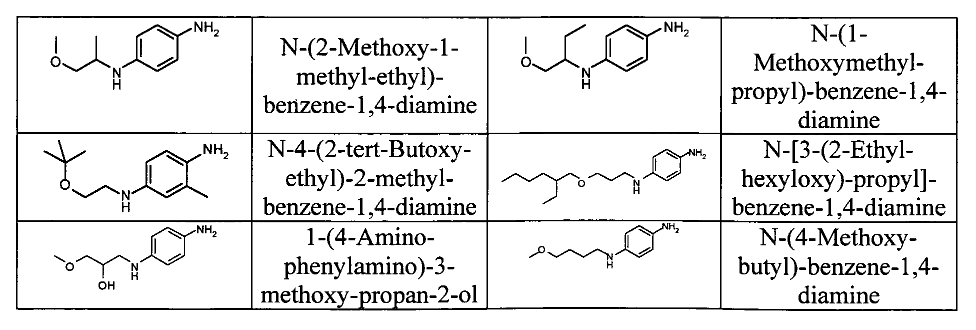

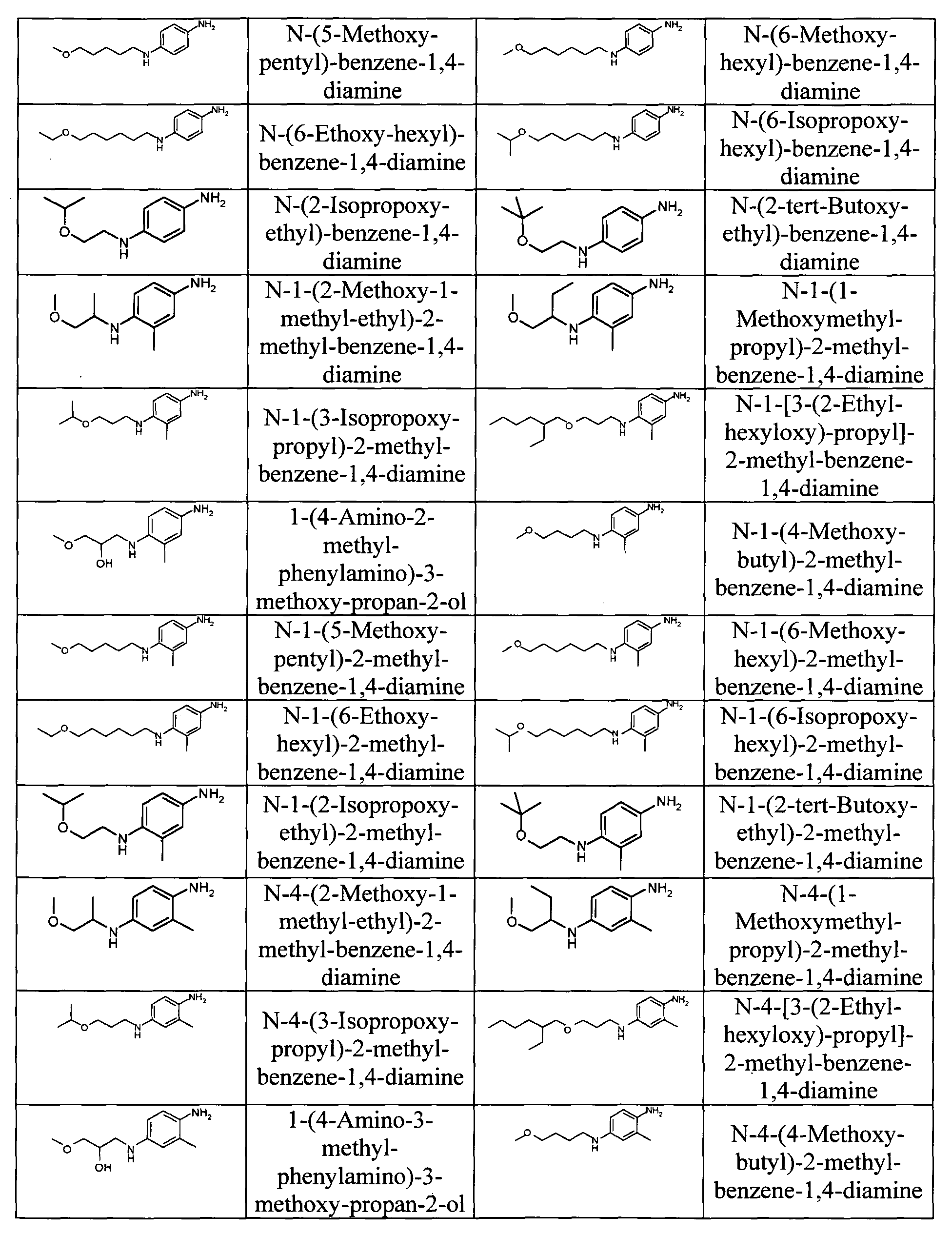

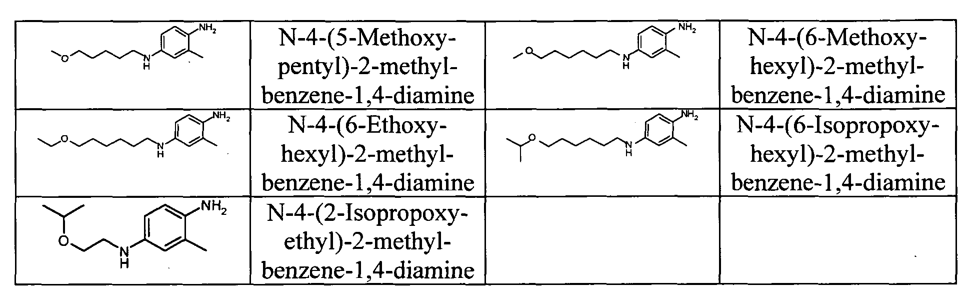

D'une manière générale, les sels d'addition utilisables pour les bases d'oxydation et les coupleurs sont notamment choisis parmi les sels d'addition avec un acide, tels que les chlorhydrates, les bromhydrates, les sulfates, les citrates, les succinates, les tartrates, les lactates, les tosylates, les benzènesulfonates, les phosphates et les acétates.

Les composés de formule (I) selon la présente demande peuvent être préparés de façon générale suivant une méthode qui comprend les étapes suivantes :
- substitution nucléophile de l'halogène en position para- dans le dérivé de para-halogénonitrobenzène, par une amine primaire de formule RNH₂ en présence d'une base, R étant défini précédemment ;
- réduction de la fonction nitro du composé obtenu à l'étape précédente en fonction amine pour obtenir le composé de formule (I).

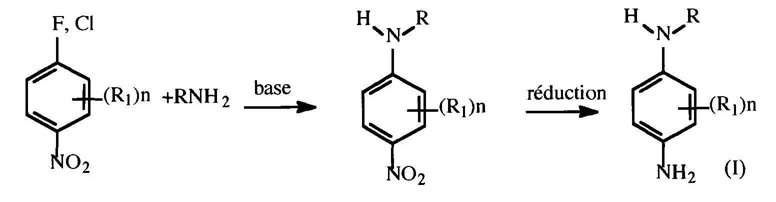

La première étape de synthèse est décrite dans les revues scientifiques Synthesis 1990 (12), 1147-1148 et Synth Commun 1990, 20(22), 3537-3545.

La deuxième étape est une étape de réduction classique, par exemple en effectuant une réaction d'hydrogénation par catalyse hétérogène en présence de Pd/C, Pd(II)/C, Ni de Raney ou encore en effectuant une réaction de réduction par un métal, par exemple par du zinc, fer, étain... (Advanced Organic Chemistry, 4^{th} edition, 1992, J. MARCH, WILEY Interscience ; Reduction in Organic Chemistry, M. Hudlicky, Ellis Honwood series Chemical Science).

La présente demande concerne aussi les composés nitrés de formule (II) et les procédés de préparation des composés para-phénylènediamines secondaires de formule (I), dans lesquels on effectue une étape de réduction du composé nitré de formule (II) correspondant. 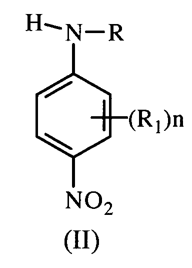 dans laquelle :
R représente un radical (alcoxy en (C₁-C₁₀) linéaire)alkyl en (C₄-C₁₀) linéaire, un radical (alcoxy en (C₁-C₁₀) linéaire)alkyl en (C₃-C₁₀) ramifié, un radical (alcoxy en (C₃-C₁₀) ramifié)alkyl en (C₂-C₁₀), un radical (alcoxy en (C₁-C₁₀))alkyl en (C₂-C₁₀) linéaire ou ramifié substitué par au moins un groupement hydroxy, alcoxy en C₁-C₁₅, amino, monoalkyl(C₁-C₁₅)amino, alkyl(C₁-C₁₅)carbonyle, amido, alcoxy(C₁-C₁₅)carbonyle, mono ou dialkyl(C₁-C₁₅)amino carbonyle;
R₁ représente un atome d'hydrogène, un radical alkyle en C₁-C₁₅, un alcoxy en C₁-C₁₅, un (alcoxy en (C₁-C₁₅))alkyle en C₁-C₁₅, un hydroxyalkyle en C₁-C₁₅, un mono ou poly-hydroxy alkyle en C₁-C₁₅, ou un atome d'halogène;
n représente un entier variant de 1 à 4.

La présente demande porte également sur l'utilisation, pour la coloration d'oxydation capillaire, du composé de formule (I), précitée dans laquelle :
R représente un radical (alcoxy en (C₁-C₁₀) linéaire)alkyle en (C₄-C₁₀) linéaire, un radical (alcoxy en (C₁-C₁₀) linéaire)alkyle en (C₃-C₁₀) ramifié, un radical (alcoxy en (C₃-C₁₀) ramifié)alkyle en (C₂-C₁₀), un radical (alcoxy en (C₁-C₁₀))alkyle en (C₂-C₁₀) linéaire ou ramifié substitué par au moins un groupement hydroxy, alcoxy en C₁-C₁₅, amino, monoalkyl(C₁-C₁₅)amino, dialkyl(C₁-C₁₅)amino, alkyl(C₁-C₁₅)carbonyle, amido, alcoxy(C₁-C₁₅)carbonyle, mono ou dialkyl(C₁-C₁₅)amino carbonyle;
R₁ représente un atome d'hydrogène, un radical alkyle en C₁-C₁₅, un alcoxy en C₁-C₁₅, un (alcoxy en (C₁-C₁₅))alkyle en C₁-C₁₅, un hydroxyalcoxy en C₁-C₁₅, un (mono ou poly)hydroxy alkyle en C₁-C₁₅, ou un atome d'halogène;
n représente un entier variant de 1 à 4 ;
ou ses sels d'addition.

La présente demande concerne aussi une composition cosmétique pour la teinture des fibres, de préférence les fibres kératiniques humaines telles que les cheveux, comprenant, dans un milieu approprié pour la teinture, au moins un composé de formule générale (I), tel qu'il vient d'être décrit.
De préférence, la composition comprend le composé de formule (I) choisi parmi les composés suivants :
-N-(2-Méthoxy-1-méthyl-éthyl)-benzène-1,4-diamine ;
-N-(1-Méthoxyméthyl-propyl)-benzène-1,4-diamine ;
-N-(3-Isopropoxy-propyl)-benzène-1,4-diamine ;
-N-[3-(2-Ethyl-hexyloxy)-propyl]-benzène-1,4-diamine ;
-1-(4-Amino-phénylamino)-3-méthoxy-propan-2-ol ;
-N-(4-Méthoxy-butyl)-benzène-1,4-diamine ;
-N-(5-Méthoxy-pentyl)-benzène-1,4-diamine ;
-N-(6-Méthoxy-hexyl)-benzène-1,4-diamine ;
-N-(6-Ethoxy-hexyl)-benzène-1,4-diamine ;
-N-(6-Isopropoxy-hexyl)-benzéne-1,4-diamine ;
-N-(2-Isopropoxy-éthyl)-benzène-1,4-diamine ;
-N-(2-tert-Butoxy-éthyl)-benzène-1,4-diamine ;
-N-1-(2-Méthoxy-1-méthyl-éthyl)-2-méthyl-benzène-1,4-diamine ;
-N-1-(1-Méthoxyméthyl-propyl)-2-méthyl-benzène-1,4-diamine ;
-N-1-(3-Isopropoxy-propyl)-2-méthyl-benzène-1,4-diamine ;
-N-1-[3-(2-Ethyl-hexyloxy)-propyl]-2-méthyl-benzène-1,4-diamine ;
-1-(4-Amino-2-méthyl-phénylamino)-3-méthoxy-propan-2-ol ;
-N-1-(4-Méthoxy-butyl)-2-méthyl-benzène-1,4-diamine ;
-N-1-(5-Méthoxy-pentyl)-2-méthyl-benzène-1,4-diamine ;
-N-1-(6-Méthoxy-hexyl)-2-méthyl-benzène-1,4-diamine ;
-N-1-(6-Ethoxy-hexyl)-2-méthyl-benzène-1,4-diamine ;
-N-1-(6-Isopropoxy-hexyl)-2-méthyl-benzène-1,4-diamine ;
-N-1-(2-Isopropoxy-éthyl)-2-méthyl-benzène-1,4-diamine ;
-N-1-(2-tert-Butoxy-éthyl)-2-méthyl-benzène-1,4-diamine ;
-N-4-(2-Méthoxy-1-méthyl-éthyl)-2-méthyl-benzène-1,4-diamine ;
-N-4-(1-Méthoxyméthyl-propyl)-2-méthyl-benzène-1,4-diamine ;
-N-4-(3-Isopropoxy-propyl)-2-méthyl-benzène-1,4-diamine ;
-N-4-[3-(2-Ethyl-hexyloxy)-propyl]-2-méthyl-benzène-1,4-diamine ;
-1-(4-Amino-3-méthyl-phénylamino)-3-méthoxy-propan-2-ol ;
-N-4-(4-Methoxy-butyl)-2-methyl-benzene-1,4-diamine ;
-N-4-(5-Méthoxy-pentyl)-2-methyl-benzène-1,4-diamine ;
-N-4-(6-Méthoxy-hexyl)-2-méthyl-benzène-1,4-diamine ;
-N-4-(6-Ethoxy-hexyl)-2-méthyl-benzène-1,4-diamine ;
-N-4-(6-Isopropoxy-hexyl)-2-méthyl-benzène-1,4-diamine ;
-N-4-(2-Isopropoxy-éthyl)-2-méthyl-benzène-1,4-diamine ;
-N-4-(2-tert-Butoxy-éthyl)-2-méthyl-benzène-1,4-diamine ;
et leurs sels d'addition.

De préférence, la concentration du composé de formule générale (I) est comprise entre 0,0001 et 20 %, de préférence entre 0,005 à 6% en poids par rapport au poids total de la composition.

Le milieu approprié pour la teinture est avantageusement constitué par de l'eau ou par un mélange d'eau et d'au moins un solvant organique tel que, par exemple, les alcools inférieurs en C₁-C₄, ramifiés ou non, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, le glycérol ainsi que les alcools aromatiques comme l'alcool benzylique ou le phénoxyéthanol, et leurs mélanges.

Les solvants sont, de préférence, présents dans des proportions de préférence comprises entre 1 et 40 % en poids environ par rapport au poids total de la composition tinctoriale, et encore plus préférentiellement entre 5 et 30 % en poids environ.

Avantageusement, la composition cosmétique comprend au moins un adjuvant cosmétique choisi dans le groupe formé par les agents antioxydants, les agents de pénétration, les agents séquestrants, les parfums, les tampons, les agents dispersants, les tensioactifs, les agents de conditionnement, les agents filmogènes, les polymères, les céramides, les agents conservateurs, les agents nacrants ou opacifiants, les vitamines ou provitamines.

Les adjuvants ci-dessus sont en général présents en quantité comprise pour chacun d'eux entre 0,01 et 20 % en poids par rapport au poids de la composition.

La composition selon l'invention contient de préférence au moins un coupleur d'oxydation.

Parmi les coupleurs d'oxydation, on peut notamment citer les méta-phénylènediamines, les méta-aminophénols, les méta-diphénols, les coupleurs naphtaléniques et les coupleurs hétérocycliques ainsi que leurs sels d'addition.

A titre d'exemple, on peut citer le 2-méthyl 5-aminophénol, le 5-N-(β-hydroxyéthyl)amino 2-méthyl phénol, le 6-chloro-2-méthyl-5-aminophénol, le 3-amino phénol, lé 1,3-dihydroxy benzène (ou résorcinol), le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine, la 3,5-diamino-2,6-diméthoxypyridine, le 1-N-(β-hydroxyéthyl)amino-3,4-méthylène dioxybenzène, le 2,6-bis-(β-hydroxyéthylamino)toluène et leurs sels d'addition.

Généralement la concentration du ou des coupleurs d'oxydation est comprise entre 0,0001 et 20%, de préférence entre 0,005 à 6% en poids par rapport au poids total de la composition.

La composition selon l'invention peut contenir au moins une base d'oxydation additionnelle différente du composé de formule (1).

Les bases d'oxydation classiques peuvent notamment être choisies parmi les para-phénylènediamines, les bis-phénylalkylènediamines, les para-aminophénols, les ortho-aminophénols et les bases hétérocycliques et leurs sels d'addition.

Parmi les para-phénylènediamines, on peut plus particulièrement citer à titre d'exemple, la para-phénylènediamine, la para-toluènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la 4-amino N,N-diéthyl 3-méthyl aniline, la N,N-bis-(β-hydroxyéthyl) para-phénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl para-phénylènediamine, la 2-fluoro para-phénylènediamine, la 2-isopropyl para-phénylènediamine, la N-(β-hydroxypropyl) para-phénylènediamine, la 2-hydroxyméthyl para-phénylènediamine, la N,N-diméthyl 3-méthyl para-phénylènediamine, la N,N-(éthyl, β-hydroxyéthyl) para-phénylènediamine, la N-(β,γ-dihydroxypropyl) para-phénylènediamine, la N-(4'-aminophényl) para-phénylènediamine, la N-phényl para-phénylènediamine, la 2-β-hydroxyéthyloxy para-phénylènediamine, la 2-β-acétylaminoéthyloxy para-phénylènediamine, la N-(β-méthoxyéthyl) para-phénylènediamine, la 4 aminophenyl pyrrolidine, le 2 thiényl para-phénylène diamine, le 2-β hydroxyéthylamino 5-amino toluène, la 3-hydroxy 1-(4' aminophenyl)pyrrolidine, la 6-(4-Amino-phenylamino)-hexan-1-olet leurs sels d'addition avec un acide.

Parmi les para-phénylènediamines citées ci-dessus, la para-phénylènediamine, la para-toluènediamine, la 2-isopropyl para-phénylènediamine, la 2-β-hydroxyéthyl para-phénylènediamine, la 2-β-hydroxyéthyloxy para-phénylènediamine, la 2,6-diméthyl para-phénylènediamine, la 2,6-diéthyl para-phénylènediamine, la 2,3-diméthyl para-phénylènediamine, la N,N-bis-(β-hydroxyéthyl) para-phénylènediamine, la 2-chloro para-phénylènediamine, la 2-β-acétytaminoéthytoxy para-phénylènediamine, et leurs sels d'addition avec un acide sont particulièrement préférées.

Parmi les bis-phénylalkylènediamines, on peut citer à titre d'exemple, le N,N'-bis-((β-hydroxyéthyl) N,N'-bis-(4'-aminophényl) 1,3-diamino propanol, la N,N'-bis-(β-hydroxyéthyt) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols, on peut citer à titre d'exemple, le para-aminophénol, le 4-amino 2-méthyl phénol, le 4-amino 3-méthyl phénol, le 4-amino 3-fluoro phénol, le 4-amino 3-chloro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, le 4-amino-2,6-dichlorophénol, le 4-amino-6[((5'-amino-2'-hydroxy-3'-méthyl)phényl)méthyl]-2-méthylphénol, le bis(5'-amino-2'-hydroxy)phénylméthane et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-alpyridine ; la 3,4-diamino-pyrazolo[1,5-alpyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs sels d'addition avec un acide.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthy!)amino 1-méthyl pyrazole, et leurs sels d'addition.

Généralement la concentration de la ou des bases d'oxydation additionnelle est comprise entre 0,0001 et 20%, de préférence entre 0,005 à 6% en poids par rapport au poids total de la composition.

D'une manière générale, les sels d'addition utilisables pour les bases d'oxydation et les coupleurs sont notamment choisis parmi les sels d'addition avec un acide, tels que les chlorhydrates, les bromhydrates, les sulfates, les citrates, les succinates, les tartrates, les lactates, les tosylates, les benzènesulfonates, les phosphates et les acétates.

La composition tinctoriale conforme à l'invention peut en outre contenir un ou plusieurs colorants directs pouvant notamment être choisis parmi les colorants nitrés de la série benzénique, neutres, acides ou cationiques, les colorants directs azoïques neutres, acides ou cationiques, les colorants directs quinoniques et en particulier anthraquinoniques neutres, acides ou cationiques, les colorants directs aziniques, les colorants directs méthiniques, azométhiniques, triarylméthaniques, indoaminiques et les colorants directs naturels. De préférence, la composition selon l'invention comprend au moins un colorant choisi parmi les colorants directs cationiques et les colorants directs naturels.

Parmi les colorants directs cationiques utilisables selon l'invention, on peut citer les colorants directs azoïques cationiques décrits dans les demandes de brevets WO 95/15144, WO-95/01772 et EP-714954.

Parmi ces composés on peut tout particulièrement citer les colorants suivants :
- chlorure de 1,3-diméthyl-2-[[4-(diméthylamino)phényl]azo]-1H-imidazolium,
- chlorure de 1,3-diméthyl-2-[(4-aminophényl)azo]-1H-imidazolium,
- méthylsulfate de 1-méthyl-4-[(méthylphénylhydrazono) méthyl]-pyridinium.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

Le ou les colorants directs représentent de préférence de 0,001 à 20% en poids environ du poids total de la composition prête à l'emploi et encore plus préférentiellement de 0,005 à 10% en poids environ.

Une composition tinctoriale prête à l'emploi est obtenue par ajout un ou plusieurs agents oxydants classiquement utilisés pour la teinture d'oxydation des fibres kératiniques, tels que par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases, le peroxyde d'hydrogène étant particulièrement préféré, à la composition tinctoriale telle que précédemment décrite.

Bien entendu, l'homme de l'art veillera à choisir le ou les adjuvants, précurseurs de colorants d'oxydations additionnels, coupleurs d'oxydation, colorants directs de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques autres que les diacides carboxyliques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule : 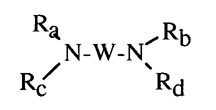 dans laquelle W est un reste propylène éventuellement substitué par un groupement hydroxyle ou un radical alkyle en C₁-C₄ ; Rₐ, R_{b}, R_{c} et R_{d}, identiques ou différents, représentent un atome d'hydrogène, un radical alkyle en C₁-C₄ ou hydroxyalkyle en C₁-C₄.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des fibres kératiniques humaines, telles que des cheveux humains.

Un autre objet de la présente demande concerne un procédé dans lequel on applique sur les fibres la composition telle que définie précédemment, et qu'on révèle la couleur à l'aide d'un agent oxydant. La couleur peut être révélée à pH acide, neutre ou alcalin. L'agent oxydant peut être ajouté à la composition de l'invention juste au moment de l'emploi. Il peut être mis en oeuvre à partir d'une composition oxydante le contenant, appliquée simultanément ou séquentiellement à la composition de l'invention.

Selon un mode de réalisation particulier, la composition selon la présente invention est mélangée, de préférence au moment de l'emploi, à une composition contenant, dans un milieu approprié pour la teinture, au moins un agent oxydant, cet agent oxydant étant présent en une quantité suffisante pour développer une coloration. Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

La composition oxydante peut renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition prête à l'emploi qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

La présente demande concerne également l'utilisation de la composition cosmétique selon l'invention comprenant, dans un milieu approprié pour la teinture, au moins un composé de formule générale (I) pour la teinture des fibres, de préférence les fibres kératiniques telles que les cheveux.

L'invention a aussi pour objet un dispositif à plusieurs compartiments ou "kit" de teinture dans lequel un premier compartiment renferme la composition tinctoriale définie ci-dessus et un deuxième compartiment renferme une composition oxydante. Ce dispositif peut être équipé d'un moyen permettant de délivrer sur les cheveux le mélange souhaité, tel que les dispositifs décrits dans le brevet FR-2 586 913 au nom de la demanderesse.

A partir de ce dispositif, il est possible de teindre les fibres kératiniques à partir d'un procédé qui comprend le mélange d'une composition tinctoriale conforme à l'invention avec un agent oxydant tel que défini précédemment, et l'application du mélange obtenu sur les fibres kératiniques pendant un temps suffisant pour développer la coloration désirée.

Les exemples qui suivent servent à illustrer l'invention sans toutefois présenter un caractère limitatif.

### EXEMPLES

### Exemple 1 : Synthèse du N-(2-Méthoxy-1-méthyl-éthyl)-benzène-1,4-diamine, dichlorhydrate (2)

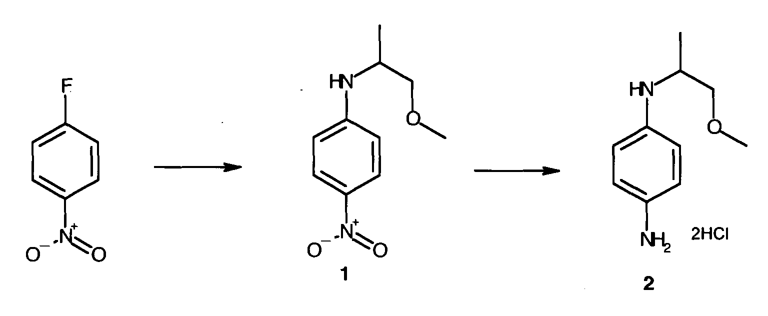

### Etape 1 : Préparation du (2-Méthoxy-1-méthyl-éthyl)-(4-nitro-phényl)-amine (1)

Dans un tricol de 150 ml, on introduit 21 g (0,15 mole) de 1-fluoro-4-nitro-benzène et 42 ml de 2-amino-1-méthoxypropane. On agite le tout 15 minutes et on porte à ébullition. Après 2 à 3 heures de réaction, le milieu réactionnel est versé sur la glace. Un précipité jaune apparaît. Celui-ci est filtré, lavé à l'eau, puis successivement avec un minimum d'éthanol, d'éther isopropylique et enfin d'éther de pétrole. 27,8 g de produit attendu ont été isolés.

| | **THEORIE** | **TROUVE** |
|---|---|---|
| C | 57.13 | 56.94 |
| H | 6.71 | 6.70 |
| N | 13.32 | 13.04 |
| O | 22.83 | 22.80 |

### Etape 2 : Préparation du N-(2-Méthoxy-1-méthyl-éthyl)-benzène-1,4-diamine, dichlorhydrate (2)

21 g (0,1 mole) de (1) sont placés dans un mélange de 42 ml d'alcool à 96°, 42 ml de cyclohexène et 4,4 ml d'eau. On y ajoute 4,2 g de palladium sur charbon 10%. Le mélange est porté au reflux 2 heures. Le catalyseur est filtré puis lavé à l'éthanol. Le filtrat est concentré à sec puis solubilisé dans 40 ml d'éthanol absolu. On refroidit à -10°C et on ajoute en une seule fois 50 ml d'éthanol chlorhydrique 7N. Le précipité blanc formé est filtré à 0°C puis on lave successivement à l'éthanol, l'acétone, l'éther isopropylique et enfin l'éther de pétrole. On obtient 2,5 g de produit attendu.

| | **THEORIE** | **TROUVE** |
|---|---|---|
| C | 47.43 | 47.30 |
| H | 7.11 | 7.12 |
| N | 11.07 | 10.94 |
| O | 6.32 | 6.57 |
| Cl | 28.06 | 28.06 |

Les spectres de RMN du proton et de masse sont conformes à la structure attendue du produit.

### Exemple 2 : Synthèse du N-(1-Méthoxyméthyl-propyl)-benzène-1,4-diamine, dichlorhydrate (4)

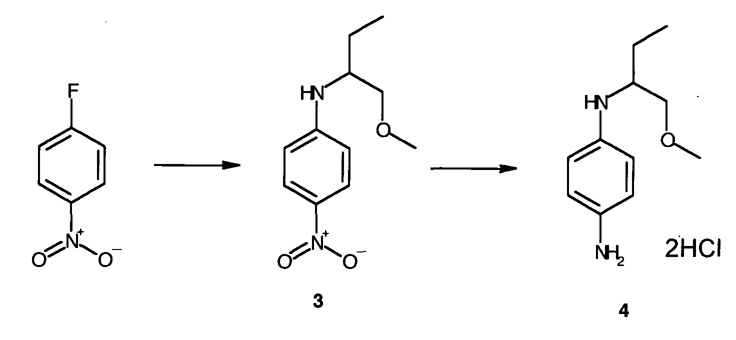

### Etape 1 : Préparation du (1-Méthoxyméthyl-propyl)-(4-nitro-phényl)-amine (3)

A 11,3 g (0,08 mole) de 1-Fluoro-4-nitro-benzène, sont ajoutés 23 g de 1-méthoxyméthylpropylamine. Le mélange est porté au reflux pendant 6h puis est versé sur la glace. Un précipité jaune apparaît ; celui-ci est filtré, lavé à l'eau, avec un minimum d'éthanol, d'éther isopropylique puis d'éther de pétrole. Après séchage, on obtient 15g de solide jaune.

| | **THEORIE** | **TROUVE** |
|---|---|---|
| C | 58.91 | 58.81 |
| H | 7.19 | 7.22 |
| N | 12.49 | 12.46 |
| O | 21.40 | 21.31 |

### Etape 2 : Préparation du N-(1-Méthoxyméthyl-propyl)-benzène-1,4-diamine, dichlorhydrate (4)

0,1 mole de (3) (22,4g) est placé dans un mélange de 42 ml d'alcool à 96°, 42 ml de cyclohexène et 4,4 ml d'eau. On y ajoute 4,2 g de palladium sur charbon 10%. Le mélange est porté au reflux 2 heures. Le catalyseur est filtré puis lavé à l'alcool. Le filtrat est concentré à sec puis solubilisé dans 40 ml d'éthanol absolu. On refroidit à -10°C et on ajoute en une seule fois 50 ml d'éthanol chlorhydrique 7N. Le précipité blanc formé est filtré à 0°C puis lavé successivement à l'éthanol, l'acétone, l'éther isopropylique et enfin l'éther de pétrole. 2,4 g de produits ont été obtenus.

| | **THEORIE, H**_{**2**}**O** | **TROUVE** |
|---|---|---|
| C | 46.32 | 46.42 |
| H | 7.77 | 7.75 |
| N | 9.82 | 9.86 |
| O | 10.22 | 10.53 |
| Cl | 24.86 | 25.43 |

Les spectres de RMN du proton et de masse sont conformes à la structure attendue du produit.

### Exemple 3 : Synthèse du N-(3-Isopropoxy-propyl)-benzène-1,4-diamine, dichlorhydrate (6)

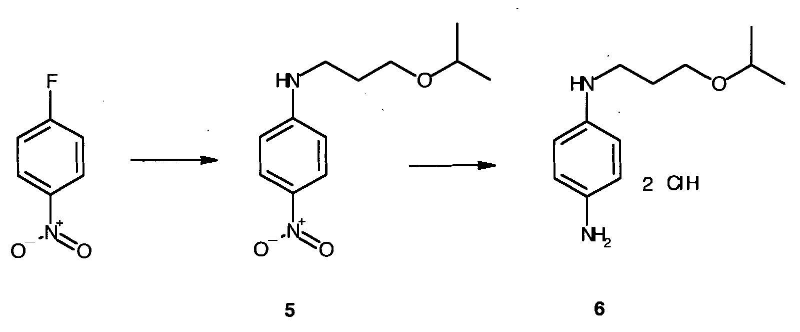

### Etape 1 : préparation du (3-Isopropoxy-propyl)-(4-nitro-phényl)-amine (5)

A 21 g (0,15 mole) de 1-Fluoro-4-nitro-benzène, sont ajoutés 42 ml de 3-isopropoxypropylamine. La réaction est exothermique et la température est maintenue à 90°C en refroidissant légèrement. Après 30 min, la réaction est totale. Le milieu est versé sur la glace ; une huile apparaît. Celle-ci est extraite à l'acétate d'éthyle ; la phase organique est lavée à l'eau et séchée sur sulfate de sodium. Après évaporation à sec, on obtient 35g d'huile jaune.

| | **THEORIE** | **TROUVE** |
|---|---|---|
| C | 60.49 | 59.61 |
| H | 7.61 | 7.66 |
| N | 11.76 | 11.74 |
| O | 20.14 | 20.64 |

### Etape 2 : Préparation du N-(3-Isopropoxy-propyl)-benzène-1,4-diamine, dichlorhydrate (6)

0,1 mole de (5) (23,8 g) est placé dans un mélange de 50 ml d'alcool à 96°, 50 ml de cyclohexène et 4,8 ml d'eau. On y ajoute 4,8 g de palladium sur charbon 10%. Le mélange est porté au reflux pendant 2 heures. Le catalyseur est filtré et le filtrat est concentré à sec puis solubilisé dans 40 ml d'éthanol absolu. On refroidit à -10°C et on ajoute en une seule fois 50 ml d'éthanol chlorhydrique 7N. Le précipité blanc formé est filtré à 0°C, puis lavé successivement à l'éthanol, l'acétone, l'éther isopropylique et enfin à l'éther de pétrole. 2,8 g de produit ont été isolés.

| | **THEORIE** | **TROUVE** |
|---|---|---|
| C | 51.25 | 51.16 |
| H | 7.83 | 7.91 |
| N | 9.96 | 9.87 |
| O | 5.69 | 5.94 |
| Cl | 25.27 | 25.33 |

Les spectres de RMN du proton et de masse sont conformes à la structure attendue du produit.

### Exemple 4 : Synthèse du N-(2-Isopropoxy-ethyl)-benzene-1,4-diamine, dichlorhydrate (8)

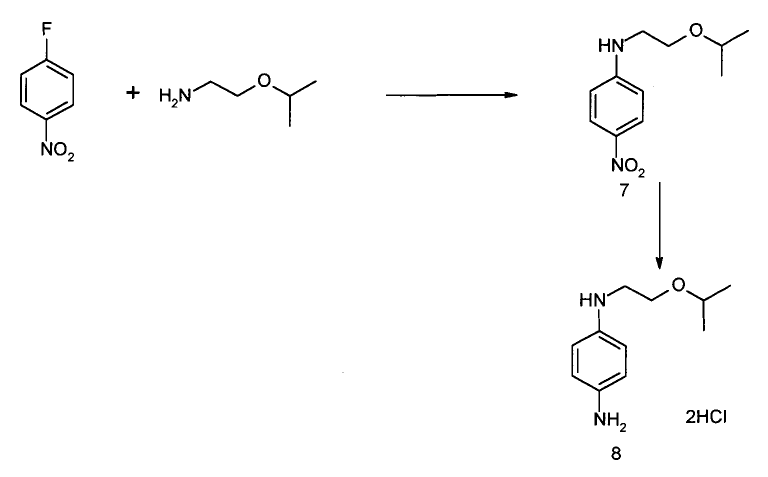

### Etape 1 : synthèse du N-(2-Isopropoxy-ethyl)-4-nitro-1-aminobenzene (7)

A une solution de 20 ml de N-méthyl-pyrrolidinone (NMP), 1,75 g de 2-aminoéthylisopropyléther et 2,35 g de K₂CO₃, on ajoute 2 g de para-fluoro-nitrobenzène. Le milieu réactionnel est chauffé à 60°C pendant 8 heures, puis, après refroidissement à la température ambiante, est versé dans un mélange eau + glace. Le précipité jaune formé est filtré, ré-empaté dans l'eau puis séché sur P₂O₅. On obtient 2,57 g de N-(2-Isopropoxy-ethyl)-4-nitro-1-aminobenzene (7).

### Etape 2 : synthèse du N-(2-Isopropoxy-ethyl)-benzene-1,4-diamine, dichlorhydrate (8)

Le N-(2-Isopropoxy-ethyl)-4-nitro-1-aminobenzene (7) précédemment obtenu est réduit avec un mélange zinc / chlorure d'ammonium/ Eau/ Ethanol bouillant. L'amine correspondante est isolée sous forme de dichlorhydrate.

Les spectres RMN du proton et de masse sont conformes à la structure attendue du produit.

### Exemple 5 : Synthèse du N-[3-(2-Ethyl-hexyloxy)-propyl]-benzene-1,4-diamine (10)

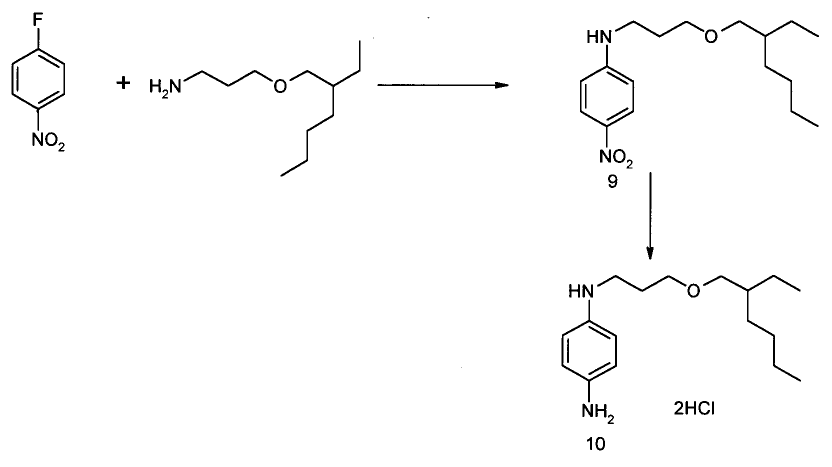

### Etape 1 : synthèse du N-{3-[(2-ethylhexyl)oxy]propyl}-N-(4-nitrophenyl)amine (9) :

A une solution de 20 ml de N-méthyl-pyrrolidinone, 3,19 g de 2-éthylhexyl-3-aminopropyléther et 2,35 g de K₂CO₃, on ajoute 2 g de para-fluoro-nitrobenzène. Le milieu réactionnel est chauffé à 60°C pendant 10 heures, puis, après refroidissement à la température ambiante, est versé dans un mélange eau + glace. Le milieu résultant est extrait à l'acétate d'éthyle puis la phase organique est concentrée par évaporation sous vide. On obtient 2,73 g de N-[2-(3-Ethyl-heptyloxy)-ethyl]-4-nitro-1-aminobenzene (9).

### Etape 2 : synthèse du N-[3-(2-Ethyl-hexyloxy)-propyl]-benzene-1,4-diamine (10) :

Le N-[2-(3-Ethyl-heptyloxy)-ethyl]-4-nitro-1-aminobenzene (9) précédemment obtenu est réduit avec un mélange zinc / chlorure d'ammonium/ Eau/ Ethanol bouillant. L'amine correspondante est isolée sous forme de dichlorhydrate.
Les spectres RMN du proton et de masse sont conformes à la structure attendue du produit.

### Exemple 6 : Synthèse du N-(4-amino-2-methoxyphenyl)-N-(3-isopropoxypropyl)amine, dihydrochloride (12)

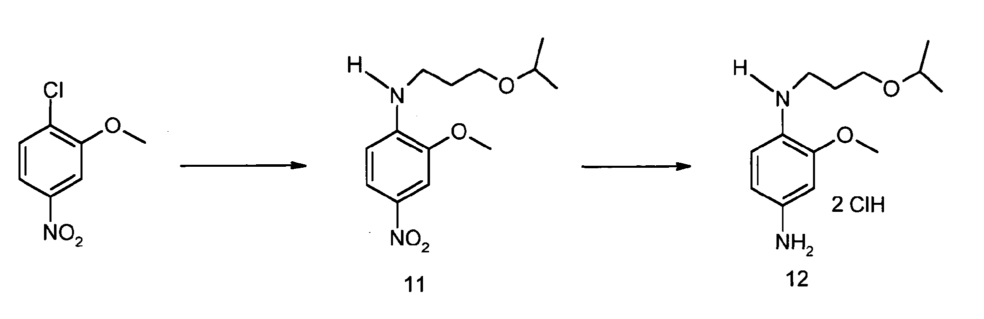

### Etape 1 : préparation du N-(3-isopropoxypropyl)-N-(2-methoxy-4-nitrophenyl)amine (11)

Dans un tricol, on introduit sous azote 1,87 g (0,01 mole) de 2-chloro-5-nitroanisole, 1,2 g (0,022 mole) de carbonate de sodium, 1,3 g (0,012 mole) de 3-isopropoxypropylamine et 10 ml de N-méthylpyrrolidinone. On chauffe jusqu'à 100 °C. Après 3 jours de réaction, le mélange réactionnel est refroidit puis 35 ml d'eau distillée est ajouté lentement avec forte agitation. Le dérivé nitré apparaît sous forme d'un semi-solide marron et est extrait au dichlorométhane puis purifié sur une colonne de silice, éluant : acétate d'éthyle / heptane : 2 / 3. On obtient 1,5 g de dérivé nitré attendu sous forme d'un solide orange.
Les spectres de RMN du proton et du ¹³C sont conformes à la structure attendue du produit.

### Etape 2: préparation du N-(4-amino-2-methoxyphenyl)-N-(3-isopropoxypropyl)amine, dihydrochloride (12)

Le N-(3-isopropoxypropyl)-N-(2-methoxy-4-nitrophenyl)amine (11) précédemment obtenu est réduit avec un mélange zinc / chlorure d'ammonium/ Eau/ Ethanol bouillant. L' amine correspondante est isolée sous forme de dichlorhydrate.

Les spectres RMN du proton et de masse sont conformes à la structure attendue du produit.

### Exemple 7 : Synthèse du 1-[(4-aminophenyl)aminol-3-methoxypropan-2-ol hydrochloride (14)

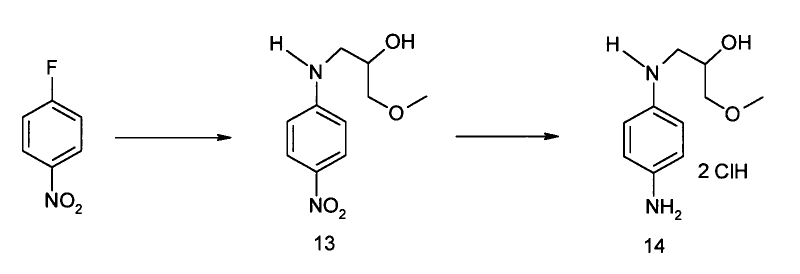

### Etape 1 : préparation du 1-methoxy-3-[(4-nitrophenyl)amino]propan-2-ol (13)

Dans un tricol, on introduit sous azote 2,82 g (0,02 mole) de 1-fluoro-4-nitrobenzène, 2,65 g (0,025 mole) de carbonate de sodium, et 5 ml de NMP. On agite puis on ajoute goutte-à-goutte 2,31 g (0,022 mole) de 1-amino-3-méthoxypropan-2-ol dans 10 ml de NMP. On chauffe jusqu'à 70 °C. Après 24 heures de réaction, le mélange réactionnel est refroidit puis 50 ml d'eau distillée est ajouté lentement avec une forte agitation. Le dérivé nitré apparaît sous la forme d'un semi-solide jaune et est extrait au dichlorométhane. La phase organique est évaporée sous vide jusqu'à l'élimination totale du NMP. On ajoute sur l'huile jaune obtenue 15 ml d'eau distillée. Un précipité jaune apparaît. Celui-ci est filtré, lavé plusieurs fois à l'eau, puis au pentane et séché sous vide. On obtient 2,9 g de dérivé nitré attendu sous forme d'une poudre jaune.

### Etape 2 : préparation du 1-[(4-aminophenyl)amino]-3-methoxypropan-2 ol dihydrochloride (14)

Le 1-methoxy-3-[(4-nitrophenyl)amino]propan-2-ol (13) précédemment obtenu est réduit avec un mélange zinc / chlorure d'ammonium/ Eau/ Ethanol bouillant. L'amine correspondante est isolée sous forme de dichlorhydrate.

Les spectres RMN du proton et de masse sont conformes à la structure attendue du produit.

### Exemple 8 : Synthèse du N-(4-aminophenyl)-N-(6-methoxyhexyl)amine dihydrochloride (17)

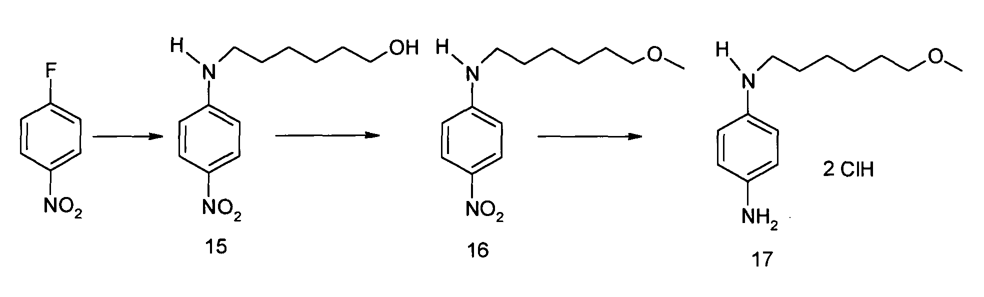

### Etape 1 : préparation du 6-[(4-nitrophenyl)amino]hexan-1-ol (15)

Dans un tricol, on introduit sous azote 2,82 g (0,02 mole) de 1-fluoro-4-nitrobenzène, 2,65 g (0,025 mole) de carbonate de sodium, et 5 ml de NMP. On agite puis on ajoute goutte-à-goutte 2,55 g (0,022 mole) de 1-amino-3-méthoxypropan-2-ol dans 15 ml de NMP. On chauffe jusqu'à 70 °C. Après 24 heures de réaction, le mélange réactionnel est refroidit puis 50 ml d'eau distillée est ajoutée lentement avec une forte agitation. Un précipité jaune apparaît. Celui-ci est filtré, lavé plusieurs fois à l'eau, puis au pentane et séché sous vide. On obtient 4,45 g de dérivé nitré attendu sous la forme d'une poudre jaune.

### Etape 2 : préparation du N-(6-methoxyhexyl)-N-(4-nitrophenyl)amine (16)

Dans un tricol, on introduit sous azote 1,19 g (0,005 mole) de dérivé nitré obtenu précédemment, et 7 ml de 1,4-dioxane. On chauffe jusqu'à 65 °C puis on introduit 1 g d'hydroxyde de potassium (85%). A cette température, on ajoute très lentement (1 goutte par minute) 1 équivalent de sulfate de diméthyle. On maintient le chauffage avec agitation pendant 2 heures. Le mélange réactionnel est refroidit puis filtré, lavé avec le dichlorométhane. Le filtrat jaune obtenu est lavé 2 fois à l'eau. La phase organique est séché puis évaporé sous vide. On obtient 1,3 g de dérivé nitré attendu sous la forme d'un produit visqueux jaune.

### Etape 3 : préparation du N-(4-aminophenyl)-N-(6-methoxyhexyl)amine dihydrochloride (17)

Le N-(6-methoxyhexyl)-N-(4-nitrophenyl)amine (16) précédemment obtenu est réduit avec un mélange zinc / chlorure d'ammonium/ Eau/ Ethanol bouillant. L'amine correspondante est isolée sous forme de dichlorhydrate.

Les spectres RMN du proton et de masse sont conformes à la structure attendue du produit.

### EXEMPLES DE TEINTURE

### Exemples 1 à 13 : Composition tinctoriale à partir du N-(2-Méthoxy-1-méthyl-éthyl)-benzène-1,4-diamine, dichlorhydrate (2)

### -Exemples 1 à 7 : Teinture en milieu acide

Les compositions tinctoriales suivantes sont préparées :

| **Exemple** | **1** | **2** | **3** | **4** | **5** | **6** | **7** |
|---|---|---|---|---|---|---|---|
| N-(2-Methoxy-1-methyl-éthyl)-benzene-1,4-diamine dichlorhydrate (2) | 10-3 mole | 10-3 mole | 10-3 mole | 10-3 mole | 10-3 mole | 10-3 mole | 10-3 mole |
| Benzene-1,3-diol | 10-3 mole | | | | | | |
| 5-Amino-2-methyl-phenol | | 10-3 mole | | | | | |
| 1H-Indol-6-ol | | | 10-3 mole | | | | |
| 2-Amino-pyridin-3-ol | | | | 10-3 mole | | | |
| 3,6-Dimethyl-1 H-pyrazolo [5,1-c][1,2 ,4 ]triazole | | | | | 10-3 mole | | |
| 2-(2,4-Diamino-phenoxy)-ethanol,chlorhydrate | | | | | | 10-3 mole 10-3 mole | |
| 3-Amino-2-chloro-6-methyl-phenol,chlorhydrate | | | | | | | 10-3 mole |
| Support de teinture (1) | (*) | (*) | (*) | (*) | (*) | (*) | (*) |
| Eau déminéralisée q.s.p. | 100g | 100g | 100g | 100g | 100g | 100g | 100g |

| | | | | | | | |
|---|---|---|---|---|---|---|---|
| (*) : support de teinture (1) pH 7 Alcool éthylique à 96° 20,8 g Métabisulfite de sodium en solution aqueuse à 35% 0,23 g M.A Sel pentasodique de l'acide diéthylène-triamine-pentaacétique en solution aqueuse à 40% 0,48 g M.A Alkyl en C₈-C₁₀ polyglucoside en solution aqueuse à 60% 3,6 g M.A Alcool benzylique 2,0 g Polyéthylène glycol à 8 motifs d'oxyde d'éthylène 3,0 g Na₂HPO₄ 0,28 g KH₂PO₄ 0,46 g | | | | | | | |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pose, les mèches sont rincées, lavées avec un shampooing standard, rincées à nouveau puis séchées.

Les nuances obtenues figurent dans le tableau ci-dessous:

| **Exemple** | **1** | **2** | **3** | **4** | **5** | **6** | **7** |
|---|---|---|---|---|---|---|---|
| **Nuance observée** | gris | violet intense | gris violet-rouge intense | gris violet intense | gris violet | bleu intense | violet-bleu intense |

### -Exemples 8 à 13 : teinture en milieu acide basique

Les compositions tinctoriales suivantes sont préparées :

| **Exemple** | **8** | **9** | **10** | **11** | **12** | **13** |
|---|---|---|---|---|---|---|
| N-(2-Methoxy-1-methy-ethyl)-benzene-1,4-diamine, dichlorhydrate (2) | 10-3 mole | 10-3 mole | 10-3 mole | 10-3 mole | 10-3 mole | 10-3 mole |
| 5-Amino-2-methyl- 10-3 phenol | 10-3 mole | | | | | |
| 1H-Indol-6-ol | | 10-3 mole | | | | |
| 2-Amino-pyridin-3-ol | | | 10-3 mole | | | |
| 3,6-Dimethyl-1 H-pyrazolo[5,1-c][1,2 ,4]triazole | | | | 10-3 mole | | |
| 2-(2,4-Diamino-phenoxy)-ethanol,chlorhydrate | | | | | 10-3 mole | |
| 3-Amino-2-chloro-6-methyl-phenol,chlorhydrate | | | | | | 10-3 mole |
| Support de teinture (2) | (*) | (*) | (*) | (*) | (*) | (*) |
| Eau déminéralisée q.s.p. | 100g | 100g | 100g | 100g | 100g | 100g |

| | | | | | | |
|---|---|---|---|---|---|---|
| (*) : support de teinture (2) pH 9,5 Alcool éthylique à 96° 20,8 g Métabisulfite de sodium en solution aqueuse à 35% 0,23 g M.A Sel pentasodique de l'acide diéthylène-triamine-pentaacétique en solution aqueuse à 40% 0,48 g M.A Alkyl en C₈-C₁₀ polyglucoside en solution aqueuse à 60% 3,6 g M.A Alcool benzylique 2,0 g Polyéthylène glycol à 8 motifs d'oxyde d'éthylène 3,0 g NH₄Cl 4,32 g Ammoniaque à 20% de NH₃ 2,94 g | | | | | | |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pause, les mèches sont rincées, lavées avec un shampooing standard, rincées à nouveau puis séchées.

Les nuances obtenues figurent dans le tableau ci-dessous :

| **Exemple** | **8** | **9** | **10** | **11** | **12** | **13** |
|---|---|---|---|---|---|---|
| **Nuance observée** | violet intense | brun rouge | brun rouge | violet-rouge chromati que | bleu intense | violet- bleu intense |

### Exemples 14 à 27 : Composition tinctoriale à partir du N-(1-Méthoxyméthyl-propyl)-benzène-1,4-diamine, dichlorhydrate (4)

### - Exemples 14 à 20 : Teinture en milieu acide

Les compositions tinctoriales suivantes sont préparées :

| **Exemple** | **14** | **15** | **16** | **17** | **18** | **19** | **20** |
|---|---|---|---|---|---|---|---|
| N-(1-Methoxymethyl-propyl)-benzene-1,4-diamine, dichlorhydrate (4) | 10-3 mole | 10-3 mole | 10-3 mole | 10-3 mole | 10-3 mole | 10-3 mole | 10-3 mole |
| Benzene-1,3-diol | 10-3 mole | | | | | | |
| 5-Amino-2-methyl-phenol | | 10-3 mole | | | | | |
| 1H-indol-6-ol | | | 10-3 mole | | | | |
| 2-Amino-pyridin-3-ol | | | | 10-3 mole | | | |
| 3,6-Dimethyl-1H-pyrazolo[5,1-c][1,2 ,4]triazole | | | | | 10-3 mole | | |
| 2-(2,4-Diamino-phenoxy)- | | | | | | 10-3 mole | |
| 3-Amino-2-chloro-6-methyl-phenol,chlorhydrate | | | | | | | 10-3 10-3 mole |
| Support de teinture | (*) | (*) | (*) | (*) | (*) | (*) | (*) |
| Eau déminéralisée | 100g | 100g | 100g | 100g | 100g | 100g | 100g |

| | | | | | | | |
|---|---|---|---|---|---|---|---|
| (*) : support de teinture (1) pH 7 Alcool éthylique à 96° 20,8 g Métabisulfite de sodium en solution aqueuse à 35% 0,23 g M.A Sel pentasodique de l'acide diéthylène-triamine-pentaacétique en solution aqueuse à 40% 0,48 g M.A Alkyl en C8-C10 polyglucoside en solution aqueuse à 60% 3,6 g M.A Alcool benzylique 2,0 g Polyéthylène glycol à 8 motifs d'oxyde d'éthylène 3,0 g Na₂HPO₄ 0,28 g KH₂PO₄ 0,46 g | | | | | | | |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pause, les mèches sont rincées, lavées avec un shampooing standard, rincées à nouveau puis séchées.

Les nuances obtenues figurent dans le tableau ci-dessous:

| **Exemple** | **14** | **15** | **16** | **17** | **18** | **19** | **20** |
|---|---|---|---|---|---|---|---|
| **Nuance observée** | gris bleu intense | violet intense | gris violet-rouge intense | gris violet intense | gris violet-bleu intense | bleu intense | violet-bleu intense |

### - Exemples 21 à 27 : teinture en milieu acide basique

Les compositions tinctoriales suivantes sont préparées :

| **Exemple** | **21** | **22** | **23** | **24** | **25** | **26** | **27** |
|---|---|---|---|---|---|---|---|
| du N-(1-Methoxymethyl-propyl)-benzene-1,4-diamine, dichlorhydrate (4) | 10-3 mole | 10-3 mole | 10-3 mole | 10-3 mole | 10-3 mole | 10-3 mole | 10-3 mole |
| Benzene-1,3-diol | 10-3 mole | | | | | | |
| 5-Amino-2-methyl-phenol | | 10-3 mole | | | | | |
| 1H-Indol-6-ol | | | 10-3 mole | | | | |
| 2-Amino-pyridin-3-ol | | | | 10-3 mole | | | |
| 3,6-Dimethyl-1 H-pyrazolo[5,1-c][1,2 ,4]triazole | | | | | 10-3 mole | | |
| 2-(2,4-Diamino-phenoxy)-ethanol,chlorhydrate | | | | | | 10-3 mole | |
| 3-Amino-2-chloro-6-methyl-phenol,chlorhydrate | | | | | | | 10-3 mole |
| Support de teinture (2) | (*) | (*) | (*) | (*) | (*) | (*) | (*) |
| Eau déminéralisée q.s.p. | 100g | 100g | 100g | 100g | 100g | 100g | 100g |

| | | | | | | | |
|---|---|---|---|---|---|---|---|
| (*) : support de teinture (2) pH 9,5 Alcool éthylique à 96° 20,8 g Métabisulfite de sodium en solution aqueuse à 35% 0,23 g M.A Sel pentasodique de l'acide diéthylène-triamine-pentaacétique en solution aqueuse à 40% 0,48 g M.A Alkyl en C₈-C₁₀ polyglucoside en solution aqueuse à 60% 3,6 g M.A Alcool benzylique 2,0 g Polyéthylène glycol à 8 motifs d'oxyde d'éthylène 3,0 g NH₄Cl 4,32 g Ammoniaque à 20% de NH₃ 2,94 g | | | | | | | |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pause, les mèches sont rincées, lavées avec un shampooing standard, rincées à nouveau puis séchées.

Les nuances obtenues figurent dans le tableau ci-dessous :

| **Exemple** | **21** | **22** | **23** | **24** | **25** | **26** | **27** |
|---|---|---|---|---|---|---|---|
| **Nuance observée** | brun brun | violet violet | rouge rouge | gris rouge | violet-rouge chromatique | bleu chromatique intense | violet-bleu intense |

### Exemples 28 à 41 : Composition tinctoriale à partir du N-(3-Isopropoxy-propyl)-benzène-1,4-diamine, dichlorhydrate (6)

### - Exemples 28 à 34 : Teinture en milieu acide

Les compositions tinctoriales suivantes sont préparées :

| **Exemple** | **28** | **29** | **30** | **31** | **32** | **33** | **34** |
|---|---|---|---|---|---|---|---|
| N-(3-Isopropoxy-propyl)-benzene-1,4-diamine dichlorhydrate (6) | 10-3 mole | 10-3 mole | 10-3 mole | 10-3 mole | 10-3 mole | 10-3 mole | 10-3 mole |
| Benzene-1,3-diol | 10-3 mole | | | | | | |
| 5-Amino-2-methyl-phenol | | 10-3 mole | | | | | |
| 1H-Indol-6-ol | | | 10-3 mole | | | | |
| 2-Amino-pyridin-3-ol | | | | 10-3 mole | | | |
| 3,6-Dimethyl-1H-pyrazolo[5,1-c][1,2 ,4]triazole | | | | | 10-3 mole mole | | |
| 2-(2,4-Diamino-phenoxy)-ethanol, chlorhydrate | | | | | | 10-3 mole | |
| 3-Amino-2-chloro-6-methyl-phenol,chlorhydrate | | | | | | | 10-3 mole |
| Support de teinture (1) | (*) | (*) | (*) | (*) | (*) | (*) | (*) |
| Eau déminéralisée q.s.p. | 100g | 100g | 100g | 100g | 100g | 100g | 100g |

| | | | | | | | |
|---|---|---|---|---|---|---|---|
| (*) : support de teinture (1) pH 7 Alcool éthylique à 96° 20,8 g Métabisulfite de sodium en solution aqueuse à 35% 0,23 g M.A Sel pentasodique de l'acide diéthylène-triamine-pentaacétique en solution aqueuse à 40% 0,48 g M.A Alkyl en C8-C10 polyglucoside en solution aqueuse à 60% 3,6 g M.A Alcool benzylique 2,0 g Polyéthylène glycol à 8 motifs d'oxyde d'éthylène 3,0 g Na₂HPO₄ 0,28 g KH₂PO₄ 0,46 g | | | | | | | |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pause, les mèches sont rincées, lavées avec un shampooing standard, rincées à nouveau puis séchées.

Les nuances obtenues figurent dans le tableau ci-dessous :

| **Exemple** | **28** | **29** | **30** | **31** | **32** | **33** | **34** |
|---|---|---|---|---|---|---|---|
| **Nuance observée** | gris intense | violet intense | gris violet intense | gris violet intense | violet-rouge intense | bleu intense | violet-bleu intense |

### -Exemples 35 à 41: teinture en milieu acide basique

Les compositions tinctoriales suivantes sont préparées :

| **Exemple** | **35** | **36** | **37** | **38** | **39** | **40** | **41** |
|---|---|---|---|---|---|---|---|
| N-(3-Isopropoxy-propyl)-benzene-1,4-diamine, dichlorhydrate (6) | 10-3 mole | 10-3 mole | 10-3 mole | 10-3 mole | 10-3 mole | 10-3 mole | 10-3 mole |
| Benzene-1,3-diol | 10-3 mole | | | | | | |
| 5-Amino-2-methyl-phenol | | 10-3 mole | | | | | |
| 1H-Indol-6-ol | | | 10-3 mole | | | | |
| 2-Amino-pyridin-3-ol | | | | mole | | | |
| 3,6-Dimethyl-1 H-pyrazolo[5,1-c][1,2 ,4]triazole | | | | | 10-3 mole | | |
| 2-(2,4-Diamino-phenoxy)-ethanol,chlorhydrate | | | | | | 10-3 mole | |
| 3-Amino-2-chloro-6-methyl-phenol,chlorhydrate | | | | | | | 10-3 mole |
| Support de teinture (2) | (*) | (*) | (*) | (*) | (*) | (*) | (*) |
| Eau déminéralisée q.s.p. | 100g | 100g | 100g | 100g | 100g | 100g | 100g |

| | | | | | | | |
|---|---|---|---|---|---|---|---|
| (*) : support de teinture (2) pH 9,5 Alcool éthylique à 96° 20,8 g Métabisulfite de sodium en solution aqueuse à 35% 0,23 g M.A Sel pentasodique de l'acide diéthylène-triamine-pentaacétique en solution aqueuse à 40% 0,48 g M.A Alkyl en C₈-C₁₀ polyglucoside en solution aqueuse à 60% 3,6 g M.A Alcool benzylique 2,0 g Polyéthylène glycol à 8 motifs d'oxyde d'éthylène 3,0 g NH₄Cl 4,32 g Ammoniaque à 20% de NH₃ 2,94 g | | | | | | | |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pause, les mèches sont rincées, lavées avec un shampooing standard, rincées à nouveau puis séchées.

Les nuances obtenues figurent dans le tableau ci-dessous :

| **Exemple** | **35** | **36** | **37** | **38** | **39** | **40** | **41** |
|---|---|---|---|---|---|---|---|
| **Nuance observée** | brun | violet intense | gris violet-rouge intense | gris | violet-rouge rouge chromatique | bleu intense | violet-bleu intense |

### Exemples 42 à 55 : Composition tinctoriale à partir du N-(2-Isopropoxy-ethyl)-benzene-1,4-diamine, dichlorhydrate (8)

### - Exemples 42 à 48 : Teinture en milieu acide

Les compositions tinctoriales suivantes sont préparées :

| **Exemple** | **42** | **43** | **44** | **45** | **46** | **47** | **48** |
|---|---|---|---|---|---|---|---|
| N-(2-ISOPROPOXY-ETHYL)-BENZENE-1,4-DIAMINE, DICHLORHYDRATE (8) | 10-3 mole | 10-3 mole | 10-3 mole | 10-3 mole | 10-3 mole | 10-3 mole | 10-3 mole |
| Benzene-1,3-diol | 10-3 mole | | | | | | |
| 5-Amino-2-methyl-phenol | | 10-3 mole | | | | | |
| 1H-Indol-6-ol | | | 10-3 mole | | | | |
| 2-Amino-pyridin-3-ol | | | | 10-3 mole | | | |
| 3,6-Dimethyl-1H-pyrazolo[5,1-c][1,2 ,4]triazole | | | | | 10-3 mole | | |
| 2-(2,4-Diamino-phenoxy)-ethanol,chlorhydrate | | | | | | 10-3 mole | |
| 3-Amino-2-chloro-6-methyl-phenol,chlorhydrate | | | | | | | 10-3 mole |
| Support de teinture (2) | (*) | (*) | (*) | (*) | (*) | (*) | (*) |
| Eau déminéralisée q.s.p. | 100g | 100g | 100g | 100g | 100g | 100g | 100g |

| | | | | | | | |
|---|---|---|---|---|---|---|---|
| (*) : support de teinture (1) pH 7 Alcool éthylique à 96° 20,8 g Métabisulfite de sodium en solution aqueuse à 35% 0,23 g M.A Sel pentasodique de l'acide diéthylène-triamine-pentaacétique en solution aqueuse à 40% 0,48 g M.A Alkyl en C8-C10 polyglucoside en solution aqueuse à 60% 3,6 g M.A Alcool benzylique 2,0 g Polyéthylène glycol à 8 motifs d'oxyde d'éthylène 3,0 g Na₂HPO₄ 0,28 g KH₂PO₄ 0,46 g | | | | | | | |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pause, les mèches sont rincées, lavées avec un shampooing standard, rincées à nouveau puis séchées.

Les nuances obtenues figurent dans le tableau ci-dessous :

| **Exemple** | **42** | **43** | **44** | **45** | **46** | **47** | **48** |
|---|---|---|---|---|---|---|---|
| **Nuance observée** | brun intense | gris violet-rouge intense | brun intense | brun rouge intense | brun rouge intense | bleu intense | violet intense |

### - Exemples 49 à 55 : teinture en milieu basique

Les compositions tinctoriales suivantes sont préparées :

| **Exemple** | **49** | **50** | **51** | **52** | **53** | **54** | **55** |
|---|---|---|---|---|---|---|---|
| N-(2-ISOPROPOXY-ETHYL)-BENZENE-14-DIAMINE, DICHLORHYDRATE (8) | 10-3 mole | 10-3 mole | 10-3 mole | 10-3 mole | 10-3 mole | 10-3 mole | 10-3 mole |
| Benzene-1, 3-diol | 10-3 mole | | | | | | |
| 5-Amino-2-methyl-phenol | | 10-3 mole | | | | | |
| 1H-Indol-6-ol | | | 10-3 mole | | | | |
| 2-Amino-pyridin-3-ol | | | | 10-3 mole | | | |
| 3,6-Dimethyl-1 H-pyrazolo[5,1-c][1,2 ,4]triazole | | | | | 10-3 mole mole | | |
| 2-(2,4-Diamino-phenoxy)-ethanol,chlorhydrate | | | | | | 10-3 mole | |
| 3-Amino-2-chloro-6-methyl-phenol,chlorhydrate | | | | | | | 10-3 mole |
| Support de teinture (1) | (*) | (*) | (*) | (*) | (*) | (*) | (*) |
| Eau déminéralisée q.s.p. | 100g | 100g | 100g | 100g | 100g | 100g | 100g |

| | | | | | | | |
|---|---|---|---|---|---|---|---|
| (*) : support de teinture (2) pH 9.5 Alcool éthylique à 96° 20,8 g Métabisulfite de sodium en solution aqueuse à 35% 0,23 g M.A Sel pentasodique de l'acide diéthylène-triamine-pentaacétique en solution aqueuse à 40% 0,48 g M.A Alkyl en C₈-C₁₀ polyglucoside en solution aqueuse à 60% 3,6 g M.A Alcool benzylique 2,0 g Polyéthylène glycol à 8 motifs d'oxyde d'éthylène 3,0 g NH₄Cl 4,32 g Ammoniaque à 20% de NH₃ 2,94 g | | | | | | | |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pause, les mèches sont rincées, lavées avec un shampooing standard, rincées à nouveau puis séchées.

Les nuances obtenues figurent dans le tableau ci-dessous :

| **Exemple** | **49** | **50** | **51** | **52** | **53** | **54** | **55** |
|---|---|---|---|---|---|---|---|
| **Nuance observée** | brun orangé | violet-rouge | orangé | rouge | rouge | bleu intense | violet-bleu intense |

### Exemples 56 à 69 : Composition tinctoriale à partir du N-[3-(2-Ethyl-hexyloxy)-propyl]-benzene-1,4-diamine (10)

### - Exemples 56 à 62 : Teinture en milieu acide

Les compositions tinctoriales suivantes sont préparées :

| Exemple | 56 | 57 | 58 | 59 | 60 | 61 | 62 |
|---|---|---|---|---|---|---|---|
| N-[3-(2-Ethyl-hexyloxy)-propyl]-benzene-1,4-diamine | **10-3 mole** | **10-3 mole** | **10-3 mole** | **10-3 mole** | **10-3 mole** | **10-3 mole** | **10-3 mole** |
| Benzene-1,3-diol | 10-3 mole | | | | | | |
| 5-Amino-2-methyl-phenol | | 10-3 mole | | | | | |
| 1H-Indol-6-ol | | | 10-3 mole | | | | |
| 2-Amino-pyridin-3-ol | | | | 10-3 mole | | | |
| 3,6-Dimethyl-1 H-pyrazolo[5,1-c][1,2 ,4]triazole | | | | | 10-3 mole | | |
| 2-(2,4-Diamino-phenoxy)-ethanol,chlorhydrate | | | | | | 10-3 mole | |
| 3-Amino-2-chloro-6-methyl-phenol,chlorhydrate | | | | | | | 10-3 mole |
| Support de teinture (2) | (*) | (*) | (*) | (*) | (*) | (*) | (*) |
| Eau déminéralisée q.s.p. | 100g | 100g | 100g | 100g | 100g | 100g | 100g |

| | | | | | | | |
|---|---|---|---|---|---|---|---|
| (*) : support de teinture (1) pH 7 Alcool éthylique à 96° 20,8 g Métabisulfite de sodium en solution aqueuse à 35% 0,23 g M.A Sel pentasodique de l'acide diéthylène-triamine-pentaacétique en solution aqueuse à 40% 0,48 g M.A Alkyl en C₈-C₁₀ polyglucoside en solution aqueuse à 60% 3,6 g M.A Alcool benzylique 2,0 g Polyéthylène glycol à 8 motifs d'oxyde d'éthylène 3,0 g Na₂HPO₄ 0,28 g KH₂PO₄ 0,46 g | | | | | | | |

Au moment de l'emploi, chaque composition est mélangée avec un poids égal d'eau oxygénée à 20 volumes (6% en poids). On obtient un pH final de 7.

Chaque mélange obtenu est appliqué sur des mèches de cheveux gris à 90 % de blancs. Après 30 minutes de pause, les mèches sont rincées, lavées avec un shampooing standard, rincées à nouveau puis séchées.

Les nuances obtenues figurent dans le tableau ci-dessous :

| **Exemple** | **56** | **57** | **58** | **59** | **60** | **61** | **62** |
|---|---|---|---|---|---|---|---|
| **Nuance observée** | brun intense | gris violet intense | gris intense | brun intense | brun rouge intense | gris violet bleu intense | gris violet intense |

### - Exemples 63 à 69 : teinture en milieu basique

Les compositions tinctoriales suivantes sont préparées :

| **Exemple** | **63** | **64** | **65** | **66** | **67** | **68** | **69** |
|---|---|---|---|---|---|---|---|
| N-[3-(2-Ethyl-hexyloxy)-propyl]-benzene-1,4-diamine (10) | 10-3 mole | 10-3 mole | 10-3 mole | 10-3 mole | 10-3 mole | 10-3 mole | 10-3 mole |
| **Benzene-1,3-diol** | 10-3 mole | | | | | | |
| **5-Amino-2-methyl-phenol** | | 10-3 mole | | | | | |
| **1 H-Indol-6-ol** | | | 10-3 mole | | | | |
| **2-Amino-pyridin-3-ol** | | | | 10-3 mole | | | |
| **3,6-Dimethyl-1H-pyrazolo[5,1- c][1,2 ,4]triazole** | | | | | 10-3 mole | | |
| **2-(2,4-Diamino-phenoxy)- ethanol,chlorhydrate** | | | | | | 10-3 mole | |
| **3-Amino-2-chloro-6-methyl- phenol,chlorhydrate** | | | | | | | 10-3 mole |
| **Support de teinture (1)** | (*) | (*) | (*) | (*) | (*) | (*) | (*) |
| **Eau déminéralisée q.s.p.** | 100g | 100g | 100g | 100g | 100g | 100g | 100g |

| | | | | | | | |
|---|---|---|---|---|---|---|---|
| (*) : support de teinture (2) pH 9.5 Alcool éthylique à 96° 20,8 g Métabisulfite de sodium en solution aqueuse à 35% 0,23 g M.A Sel pentasodique de l'acide diéthylène-triamine-pentaacétique en solution aqueuse à 40% 0,48 g M.A Alkyl en C₈-C₁₀ polyglucoside en solution aqueuse à 60% 3,6 g M.A Alcool benzylique 2,0 g Polyéthylène glycol à 8 motifs d'oxyde d'éthylène 3,0 g NH₄Cl 4,32 g Ammoniaque à 20% de NH₃ 2,94 g | | | | | | | |

Au moment de l'emploi, chaque composition est mélangée avec un poids égal d'eau oxygénée à 20 volumes (6% en poids). On obtient un pH final de 9,5.

Chaque mélange obtenu est appliqué sur des mèches de cheveux gris à 90 % de blancs. Après 30 minutes de pause, les mèches sont rincées, lavées avec un shampooing standard, rincées à nouveau puis séchées.

Les nuances obtenues figurent dans le tableau ci-dessous :

| **Exemple** | **63** | **64** | **65** | **66** | **67** | **68** | **69** |
|---|---|---|---|---|---|---|---|
| **Nuance observée** | **brun intense** | **gris violet- rouge intense** | **brun brun rouge intense** | **rouge intense** | **rouge intense** | **gris violet- bleu intense** | **violet intense** |

## Revendications

1. Composé **caractérisé en ce qu'**il s'agit d'une para-phénylènediamine secondaire de formule générale (1) : 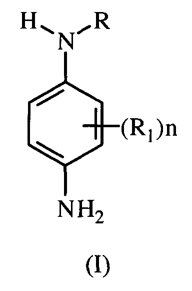 dans laquelle :
R représente un radical (alcoxy en (C₁-C₁₀) linéaire)alkyle en (C₄-C₁₀) linéaire, un radical (alcoxy en (C₁-C₁₀) linéaire)alkyle en (C₃-C₁₀) ramifié, un radical (alcoxy en (C₃-C₁₀) ramifié)alkyle en (C₂-C₁₀), un radical (alcoxy en (C₁-C₁₀))alkyle en (C₂-C₁₀) linéaire ou ramifié substitué par au moins un groupement hydroxy, alcoxy en C₁-C₁₅, amino, monoalkyl(C₁-C₁₅)amino, dialkyl(C₁-C₁₅)amino, alkyl(C₁-C₁₅)carbonyle, amido, alcoxy(C₁-C₁₅)carbonyle, mono ou dialkyl(C₁-C₁₅)amino carbonyle;
R₁ représente un atome d'hydrogène, un radical alkyle en C₁-C₁₅, un alcoxy en C₁-C₁₅, un (alcoxy en (C₁-C₁₅))alkyle en C₁-C₁₅, un hydroxyalcoxy en C₁-C₁₅, un (mono ou poly)hydroxy alkyle en C₁-C₁₅, ou un atome d'halogène;
n représente un entier variant de 1 à 4 ;
ou ses sels d'addition ;
à l'exception des composés suivants :
N-(3-isopropoxypropyl)-benzène 1,4-diamine;
N-(1-méthylpropoxypropyl)-benzène 1,4-diamine.

2. Composé selon la revendication 1, **caractérisé en ce que** le groupement R de la formule (I) représente un radical (alcoxy en (C₁-C₅) linéaire)alkyle en (C₄-C₈) linéaire, un radical (alcoxy en (C₁-C₅) linéaire)alkyle en (C₃-C₇) ramifié, un radical (alcoxy en (C₃-C₇) ramifié)alkyle en (C₂-C₆), un radical (alcoxy en (C₁-C₆))alkyle en (C₂-C₆) linéaire ou ramifié substitué par au moins un groupement hydroxy, alcoxy en C₁-C₅, amino, monoalkyl(C₁-C₅)amino, dialkyl(C₁-C₅)amino, alkyl(C₁-C₅)carbonyle, amido, alcoxy(C₁-C₅)carbonyle, mono ou dialkyl(C₁-C₅)amino carbonyle.

3. Composé selon la revendication 1 ou 2, **caractérisé en ce que** le groupement R₁ de la formule (I) représente un atome d'hydrogène, un radical alkyle en C₁-C₅, un alcoxy en C₁-C₅, un (alcoxy en (C₁-C₅))alkyle en C₁-C₅, un hydroxyalcoxy en C₁-C₅, un mono ou poly-hydroxy alkyle en C₁-C₅, ou un atome de chlore ou de brome.

4. Composé selon l'une quelconque des revendications 1 à 3, **caractérisé en ce que** le composé de formule (I) est choisi parmi les composés suivants:
-N-(2-Méthoxy-1-méthyl-éthyl)-benzène-1,4-diamine ;
-N-(1-Méthoxyméthyl-propyl)-benzène-1,4-diamine ;
-N-[3-(2-Ethyl-hexyloxy)-propyl]-benzène-1,4-diamine ;
-1-(4-Amino-phénylamino)-3-méthoxy-propan-2-ol ;
-N-(4-Méthoxy-butyl)-benzène-1,4-diamine ;
-N-(5-Méthoxy-pentyl)-benzène-1,4-diamine ;
-N-(6-Méthoxy-hexyl)-benzène-1,4-diamine ;
-N-(6-Ethoxy-hexyl)-benzène-1,4-diamine ;
-N-(6-Isopropoxy-hexyl)-benzéne-1,4-diamine ;
-N-(2-Isopropoxy-éthyl)-benzène-1,4-diamine ;
-N-(2-tert-Butoxy-éthyl)-benzène-1,4-diamine ;
-N-1-(2-Méthoxy-1-méthyl-éthyl)-2-méthyl-benzène-1,4-diamine ;
-N-1-(1-Méthoxyméthyl-propyl)-2-méthyl-benzène-1,4-diamine ;
-N-1-(3-Isopropoxy-propyl)-2-méthyl-benzène-1,4-diamine ;
-N-1-[3-(2-Ethyl-hexyloxy)-propyl]-2-méthyl-benzène-1,4-diamine ;
-1-(4-Amino-2-méthyl-phénylamino)-3-méthoxy-propan-2-ol ;
-N-1-(4-Méthoxy-butyl)-2-méthyl-benzène-1,4-diamine ;
-N-1-(5-Méthoxy-pentyl)-2-méthyl-benzène-1,4-diamine ;
-N-1-(6-Méthoxy-hexyl)-2-méthyl-benzène-1,4-diamine ;
-N-1-(6-Ethoxy-hexyl)-2-méthyl-benzène-1,4-diamine ;
-N-1-(6-Isopropoxy-hexyl)-2-méthyl-benzène-1,4-diamine ;
-N-1-(2-Isopropoxy-éthyl)-2-méthyl-benzène-1,4-diamine ;
-N-1-(2-tert-Butoxy-éthyl)-2-méthyl-benzène-1,4-diamine ;
-N-4-(2-Méthoxy-1-méthyl-éthyl)-2-méthyl-benzène-1,4-diamine ;
-N-4-(1-Méthoxyméthyl-propyl)-2-méthyl-benzène-1,4-diamine ;
-N-4-(3-Isopropoxy-propyl)-2-méthyl-benzène-1,4-diamine ;
-N-4-[3-(2-Ethyl-hexyloxy)-propyl]-2-méthyl-benzène-1,4-diamine ;
-1-(4-Amino-3-méthyl-phénylamino)-3-méthoxy-propan-2-ol ;
-N-4-(4-Methoxy-butyl)-2-methyl-benzene-1,4-diamine ;
-N-4-(5-Méthoxy-pentyl)-2-methyl-benzène-1,4-diamine ;
-N-4-(6-Méthoxy-hexyl)-2-méthyl-benzène-1,4-diamine ;
-N-4-(6-Ethoxy-hexyl)-2-méthyl-benzène-1,4-diamine ;
-N-4-(6-Isopropoxy-hexyl)-2-méthyl-benzène-1,4-diamine ;
-N-4-(2-Isopropoxy-éthyl)-2-méthyl -benzène- 1,4-di amine ;
-N-4-(2-tert-Butoxy-éthyl)-2-méthyl -benzène- 1,4-di amine ;
et leurs sels d'addition.

5. Composé selon l'une quelconque des revendications 1 à 4, **caractérisé en ce que** les sels d'addition avec un acide des para-phénylènediamines secondaires de formule générale (I) sont choisis parmi les chlorhydrates, les bromhydrates, les sulfates, les citrates, les succinates, les tartrates, les lactates, les tosylates, les benzènesulfonates, les phosphates et les acétates.

6. Composé nitré **caractérisé en ce qu'**il possède la structure suivante (II) : 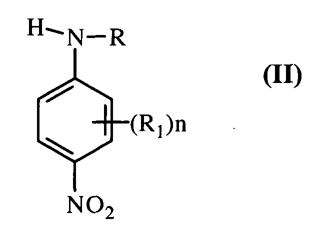 dans laquelle :
R représente un radical (alcoxy en (C₁-C₁₀) linéaire)alkyl en (C₄-C₁₀) linéaire, un radical (alcoxy en (C₁-C₁₀) linéaire)alkyl en (C₃-C₁₀) ramifié, un radical (alcoxy en (C₃-C₁₀) ramifié)alkyl en (C₂-C₁₀), un radical (alcoxy en (C₁-C₁₀))alkyl en (C₂-C₁₀) linéaire ou ramifié substitué par au moins un groupement hydroxy, alcoxy en C₁-C₁₅, amino, monoalkyl(C₁-C₁₅)amino, alkyl(C₁-C₁₅)carbonyle, amido, alcoxy(C₁-C₁₅)carbonyle, mono ou dialkyl(C₁-C₁₅)amino carbonyle;
R₁ représente un atome d'hydrogène, un radical alkyle en C₁-C₁₅, un alcoxy en C₁-C₁₅, un (alcoxy en (C₁-C₁₅))alkyle en C₁-C₁₅, un hydroxyalkyle en C₁-C₁₅, un mono ou poly-hydroxy alkyle en C₁-C₁₅, ou un atome d'halogène;
n représente un entier variant de 1 à 4.

7. Procédé de préparation du composé de formule (I) tel que défini dans les revendications 1 à 5, **caractérisé par le fait qu'**on effectue une étape de réduction d'un composé nitré de formule (II) tel que défini à la revendication 6.

8. Utilisation du composé de formule (I) : 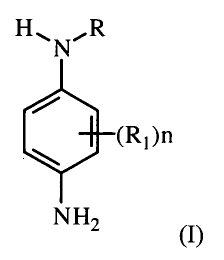 dans laquelle :
R représente un radical (alcoxy en (C₁-C₁₀) linéaire)alkyle en (C₄-C₁₀) linéaire, un radical (alcoxy en (C₁-C₁₀) linéaire)alkyle en (C₃-C₁₀) ramifié, un radical (alcoxy en (C₃-C₁₀) ramifié)alkyle en (C₂-C₁₀), un radical (alcoxy en (C₁-C₁₀))alkyle en (C₂-C₁₀) linéaire ou ramifié substitué par au moins un groupement hydroxy, alcoxy en C₁-C₁₅, amino, monoalkyl(C₁-C₁₅)amino, dialkyl(C₁-C₁₅)amino, alkyl(C₁-C₁₅)carbonyle, amido, alcoxy(C₁-C₁₅)carbonyle, mono ou dialkyl(C₁-C₁₅)amino carbonyle;
R₁ représente un atome d'hydrogène, un radical alkyle en C₁-C₁₅, un alcoxy en C₁-C₁₅, un (alcoxy en (C₁-C₁₅))alkyle en C₁-C₁₅, un hydroxyalcoxy en C₁-C₁₅, un (mono ou poly)hydroxy alkyle en C₁-C₁₅, ou un atome d'halogène;
n représente un entier variant de 1 à 4 ;
ou ses sels d'addition, tel que défini dans l'une quelconque des revendications 2, 3 et 5, pour la coloration d'oxydation capillaire.

9. Composition cosmétique pour la teinture des fibres kératiniques, de préférence les fibres kératiniques humaines, telles que les cheveux, comprenant, dans un milieu approprié pour la teinture, au moins un composé de formule générale (I) : 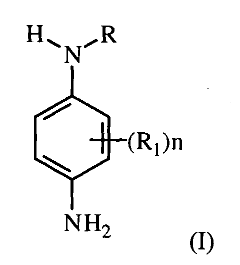 dans laquelle :
R représente un radical (alcoxy en (C₁-C₁₀) linéaire)alkyle en (C₄-C₁₀) linéaire, un radical (alcoxy en (C₁-C₁₀) linéaire)alkyle en (C₃-C₁₀) ramifié, un radical (alcoxy en (C₃-C₁₀) ramifié)alkyle en (C₂-C₁₀), un radical (alcoxy en (C₁-C₁₀))alkyle en (C₂-C₁₀) linéaire ou ramifié substitué par au moins un groupement hydroxy, alcoxy en C₁-C₁₅, amino, monoalkyl(C₁-C₁₅)amino, dialkyl(C₁-C₁₅)amino, alkyl(C₁-C₁₅)carbonyle, amido, alcoxy(C₁-C₁₅)carbonyle, mono ou dialkyl(C₁-C₁₅)amino carbonyle;
R₁ représente un atome d'hydrogène, un radical alkyle en C₁-C₁₅, un alcoxy en C₁-C₁₅, un (alcoxy en (C₁-C₁₅))alkyle en C₁-C₁₅, un hydroxyalcoxy en C₁-C₁₅, un (mono ou poly)hydroxy alkyle en C₁-C₁₅, ou un atome d'halogène;
n représente un entier variant de 1 à 4 ;
ou ses sels d'addition, tel que défini dans l'une quelconque des revendications 2, 3 et 5.

10. Composition selon la revendication précédente, **caractérisée en ce que** le composé de formule (I) est choisi parmi les composés suivants :
-N-(2-Méthoxy-1-méthyl-éthyl)-benzène-1,4-diamine ;
-N-(1-Méthoxyméthyl-propyl)-benzène-1,4-diamine ;
-N-(3-Isopropoxy-propyl)-benzène-1,4-diamine ;
-N-[3-(2-Ethyl-hexyloxy)-propyl]-benzène-1,4-diamine ;
-1-(4-Amino-phénylamino)-3-méthoxy-propan-2-ol ;
-N-(4-Méthoxy-butyl)-benzène-1,4-diamine ;
-N-(5-Méthoxy-pentyl)-benzène-1,4-diamine ;
-N-(6-Méthoxy-hexyl)-benzène-1,4-diamine ;
-N-(6-Ethoxy-hexyl)-benzène-1,4-diamine ;
-N-(6-Isopropoxy-hexyl)-benzéne-1,4-diamine ;
-N-(2-Isopropoxy-éthyl)-benzène-1,4-diamine ;
-N-(2-tert-Butoxy-éthyl)-benzène-1,4-diamine ;
-N-1-(2-Méthoxy-1-méthyl-éthyl)-2-méthyl-benzène-1,4-diamine ;
-N-1-(1-Méthoxyméthyl-propyl)-2-méthyl-benzène-1,4-diamine ;
-N-1-(3-Isopropoxy-propyl)-2-méthyl-benzène-1,4-diamine ;
-N-1-[3-(2-Ethyl-hexyloxy)-propyl]-2-méthyl-benzène-1,4-diamine ;
-1-(4-Amino-2-méthyl-phénylamino)-3-méthoxy-propan-2-ol ;
-N-1-(4-Méthoxy-butyl)-2-méthyl-benzène-1,4-diamine ;
-N-1-(5-Méthoxy-pentyl)-2-méthyl-benzène-1,4-diamine ;
-N-1-(6-Méthoxy-hexyl)-2-méthyl-benzène-1,4-diamine ;
-N- 1 -(6-Ethoxy-hexyl)-2-méthyl -benzène- 1,4-di amine ;
-N-1-(6-Isopropoxy-hexyl)-2-méthyl-benzène-1,4-diamine ;
-N-1-(2-Isopropoxy-éthyl)-2-méthyl-benzène-1,4-diamine ;
-N-1-(2-tert-Butoxy-éthyl)-2-méthyl-benzène-1,4-diamine ;
-N-4-(2-Méthoxy-1-méthyl-éthyl)-2-méthyl-benzène-1,4-diamine ;
-N-4-(1-Méthoxyméthyl-propyl)-2-méthyl-benzène-1,4-diamine ;
-N-4-(3-Isopropoxy-propyl)-2-méthyl-benzène-1,4-diamine ;
-N-4-[3-(2-Ethyl-hexyloxy)-propyl]-2-méthyl-benzène-1,4-diamine ;
-1-(4-Amino-3-méthyl-phénylamino)-3-méthoxy-propan-2-ol ;
-N-4-(4-Methoxy-butyl)-2-methyl-benzene-1,4-diamine ;
-N-4-(5-Méthoxy-pentyl)-2-methyl-benzène-1,4-diamine ;
-N-4-(6-Méthoxy-hexyl)-2-méthyl-benzène-1,4-diamine ;
-N-4-(6-Ethoxy-hexyl)-2-méthyl-benzène-1,4-diamine ;
-N-4-(6-Isopropoxy-hexyl)-2-méthyl-benzène-1,4-diamine ;
-N-4-(2-Isopropoxy-éthyl)-2-méthyl-benzène-1,4-diamine ;
-N-4-(2-tert-Butoxy-éthyl)-2-méthyl-benzène-1,4-diamine ;
et leurs sels d'addition.

11. Composition selon la revendication 9 ou 10, **caractérisée en ce que** la concentration du composé de formule générale (I) est comprise entre 0,0001 et 20%, de préférence entre 0,005 à 6% en poids par rapport au poids total de la composition

12. Composition selon l'une quelconque des revendications 9 à 11, **caractérisée en ce que** le milieu approprié pour la teinture est constitué par de l'eau ou par un mélange d'eau et d'au moins un solvant organique choisi parmi les alcools inférieurs en C₁-C₄, ramifiés ou non ; les polyols et éthers de polyols; les alcools aromatiques et leurs mélanges.

13. Composition selon l'une quelconque des revendications 9 à 12, **caractérisée en ce qu'**elle comprend au moins un adjuvant cosmétique choisi parmi les agents antioxydants, les agents de pénétration, les agents séquestrants, les parfums, les tampons, les agents dispersants, les tensioactifs, les agents conditionneurs, les agents filmogènes, les polymères, les céramides, les agents conservateurs, les agents nacrants
ou opacifiants, les vitamines ou provitamines.

14. Composition selon la revendication 13 **caractérisée en ce que** la quantité en adjuvant cosmétique est comprise pour chacun d'eux entre 0,01 et 20 % en poids par rapport au poids total de la composition.

15. Composition selon l'une quelconque des revendications 9 à 14, **caractérisée en ce que** la composition comprend au moins un coupleur d'oxydation choisi parmi les méta-phénylènediamines, les méta-aminophénols, les méta-diphénols, les coupleurs naphtaléniques, les coupleurs hétérocycliques ainsi que leurs sels d'addition.

16. Composition selon la revendication 15, **caractérisée en ce que** la concentration du ou des coupleurs est comprise entre 0,0001 et 20 % en poids par rapport au poids total de la composition.

17. Composition selon l'une quelconque des revendications 9 à 16, **caractérisée en ce que** la composition comprend au moins une base d'oxydation additionnelle différente du composé de formule (I) choisie parmi les para-phénylènediamines, les bis-phénylalkylènediamines, les para-aminophénols, les ortho-aminophénols, les bases hétérocycliques et leurs sels d'addition.

18. Composition selon la revendication 17, **caractérisée en ce que** la concentration de la ou des bases d'oxydation additionnelle est comprise entre 0,0001 et 20% en poids par rapport au poids total de la composition.

19. Composition selon l'une quelconque des revendications 9 à 18, **caractérisée en ce qu'**elle renferme un ou plusieurs colorants directs naturels ou cationiques.

20. Procédé de teinture des fibres kératiniques et en particulier des fibres kératiniques humaines telles que les cheveux, **caractérisée par le fait qu'**on applique sur lesdites fibres au moins une composition selon l'une quelconque des revendications 9 à 19, pendant une durée suffisante pour développer la coloration désirée en présence d'un agent oxydant.

21. Composition prête à l'emploi, **caractérisée en ce qu'**elle comprend dans une composition tinctoriale telle que définie dans l'une quelconque des revendications 9 à 19 au moins un agent oxydant choisi parmi le peroxyde d'hydrogène, le peroxyde d'urée, les bromates de métaux alcalins, les persels, les peracides et les enzymes oxydases, et de préférence le peroxyde d'hydrogène

22. Utilisation pour la teinture des fibres kératiniques et en particulier des fibres kératiniques humaines telles que les cheveux de la composition telle que définie dans l'une quelconque des revendications 9 à 19.

23. Dispositif à plusieurs compartiments dans lequel le premier compartiment contient une composition tinctoriale pour la teinture des fibres kératiniques telle que définie à l'une quelconque des revendications 9 à 19 et un deuxième compartiment contient un agent oxydant.
